# EUROPEAN PATENT SPECIFICATION

(11) **EP 4 268 738 B1**
(45) Date of publication and mention of the grant of the patent: **04.06.2025**
(21) Application number: 22170525.4
(22) Date of filing: 28.04.2022
(51) Int. Cl.: A61B 17/34

(54) **AN INSTRUMENT FIXATION ASSEMBLY AND A TROCAR FIXATION SYSTEM**
INSTRUMENTENBEFESTIGUNGSANORDNUNG UND TROKARBEFESTIGUNGSSYSTEM
ENSEMBLE FIXATION D'INSTRUMENT ET SYSTÉME DE FIXATION DE TROCART

(43) Date of publication of application: 01.11.2023
(73) Proprietor: Mölnlycke Health Care AB, 431 21 Mölndal (SE)
(72) Inventor: ROSENGREN, Oscar, 436 55 Hovås (SE); GUNGNER, Mattias, 441 60 Alingsås (SE)
(74) Representative: Kransell & Wennborg KB

(56) References cited:
- EP-A1- 2 965 700
- WO-A1-2020/243156
- US-A- 5 190 561
- US-A- 5 662 613
- US-A- 5 713 869
- US-A1- 2010 010 449
- US-A1- 2010 292 724
- US-A1- 2013 085 342
- US-A1- 2021 068 917

## Description

### TECHNICAL FIELD

The present disclosure relates to an instrument fixation device for holding a medical instrument in a trocar system, a trocar fixation system comprising a trocar fixation device for holding a trocar during use and an instrument fixation device and a trocar system comprising a trocar and the trocar fixation system.

### BACKGROUND

Minimal invasive surgery (MIS) is a technique for performing surgery that is associated with less pain, shorter recovery and fewer complications compared to traditional open surgery.

In an MIS procedure, a small incision is typically made through the skin of a patient, and a trocar is thereafter inserted into the incision. The trocar serves as a working channel that allows for the entry of various medical instruments and cameras into a body cavity, e.g. an abdominal cavity.

A trocar typically comprises a cannula and an obturator. The cannula is a hollow and tube-shaped shaft that is placed inside the patient to provide access to the body cavity during the MIS procedure. The obturator is a sharp tool that allows the cannula to penetrate the body cavity for initial placement. When the cannula has entered the body cavity, the obturator is removed.

During an MIS procedure, a plurality of trocars is typically utilized. Accordingly, the surgeon can utilize a variety of medical instruments and tools and alternate between the different trocars throughout the procedure. Often, the surgeon utilizes more than one surgical tool at the same time. In such scenarios, the medical staff must typically support the surgeon in fixating the trocar and the medical instruments in the trocar, and in facilitating the access to the various trocars.

During laparascopic and other minimally invasive surgical procedures, it may be desired to lock the position of the medical instruments or tools inserted into the cannula. This is particularly the case for medical instruments intended for retracting and pushing away abdominal structures to facilitate access to a particular body cavity or organ where the surgical procedure is to be performed. This may currently be done by means of a staff member holding the medical instrument in position during the procedure.

Similarly, the trocar needs to be held still in a straight or a tilted configuration in the body cavity. In order to keep a trocar in a straight or in a tilted configuration, a member of the surgical staff may need to hold the trocar in position. If the trocar is allowed to move freely within the body cavity and is repeatedly exposed to dislocation, this may cause undesired movement of the trocar inside the patient, and also increase the tension of the surgical incision and the skin area surrounding the incision. Furthermore, the surgeon must typically lean over the patient in order to access the plurality of trocars inserted into the skin of the patient. This may be challenging from an ergonomic perspective.

In view of this, it would be desirable to be able to lock and unlock the position of the medical instrument after inserting the medical instrument in the trocar system in an improved and facilitated manner, to prevent dislocation of the trocar after insertion into the body cavity and also to facilitate the access to the trocars during the MIS procedure.

Accordingly, there is a need to provide an improved and simplified means for fixating a medical instrument in a trocar system and as well fixating a trocar while also relieving the burden for the surgical staff in performing the minimal invasive surgery. Such means should provide for a simplified, and safe MIS procedure to be carried out.

US 2013/085342 A1 discloses a device for fixing a surgical instrument with respect to a surrounding trocar.

### SUMMARY

The invention is defined by independent claim 1. Further embodiments are defined by the dependent claims. No surgical methods are claimed.

In view of the above-mentioned problems, it is an object of the present disclosure to provide improvements with respect to relieving the burden for surgical staff and providing a facilitated and improved means to handle and fixate a medical instrument in a trocar and to handle and fixate a medical instrument and a trocar during minimal invasive surgery.

According to a first aspect, an instrument fixation device for holding a medical instrument in a trocar system is provided. The trocar system comprises a trocar cannula and a trocar housing. The instrument fixation device comprises a trocar housing connector adapted for connection between the instrument fixation device and the trocar housing. The instrument fixation device comprises a first side intended to face the trocar housing during use and a second side being an opposite side to the first side. The instrument fixation device comprises an instrument receiving opening extending in a longitudinal direction of the instrument fixation device between the first and the second side of the instrument fixation device. The instrument fixation device has an open configuration allowing the medical instrument to move freely in the longitudinal direction and a closed configuration wherein the medical instrument is restrained from movement in the longitudinal direction by clamping of the elongated portion of the medical instrument between a first and second opposing clamping surface of the instrument fixation device with a clamping force being directed in a transverse direction being perpendicular to the longitudinal direction. The instrument fixation device comprises an actuation member configured for shifting the configuration of the instrument fixation device between the open configuration and the closed configuration, by means of a translational movement of the actuation member. The translational movement being in the transverse direction of the instrument fixation device and wherein the closed configuration of the instrument fixation device includes a sub-set of positions with varying distances between the first and the second opposing clamping surface in each of the positions.

The present invention is based on the realization that by providing an instrument fixation device being connectable to a trocar housing and which enables medical instruments of varying sizes to be locked in a fixed position within the cannula according to the present disclose, an improved, more flexible and facilitated minimally invasive surgical procedure could be achieved for the surgeon and for the surgical staff.

The fact that the translational movement of the actuation member is in the transverse direction has been found to facilitate gripping of the instrument fixation device and handling of the actuation member, such that the configuration of the instrument fixation device may be shifted from open to closed configuration and the medical instrument may be locked in position, as seen in the longitudinal direction. Furthermore, as the translational movement of the actuation member is in the transverse direction when handling of the actuation member and shifting from the open to the closed position, the risk of accidentally rotating the trocar or displacing the trocar further into the incision, corresponding to the longitudinal direction of the instrument fixation device, is reduced and the handling of the instrument fixation device facilitated and improved.

During the surgical procedure, the trocar may be used for more than one medical instrument. One or more of the trocars may be used for a medical instrument comprising a camera for inspection during the surgical procedure. Medical instruments for clamping or cutting of tissue may also be required and scissors, retractors or clip applier may be introduced during the surgical procedures, and at a different stage of the procedure be removed for introducing another one of the instruments via the same trocar. Medical instruments may vary in size and may have a variety of different diameters, and the instrument fixation device according to the present disclosure provides flexibility and facilitates the surgical procedure by providing a locked position irrespective of the diameter of the instrument fixation device. A limitation of the flexibility in the larger size diameter range evidentially being the size of the opening in the trocar.

The actuation device is configured for gradually moving, continuously or discontinuously, the first and second opposing clamping surface(s) in the transverse direction.

The sub-sets of positions in the locked configuration may be either continuous or discontinuous positions. An actuation device and an instrument fixation device configured for gradually moving the first and/or second opposing clamping surfaces may provide a locked configuration of the instrument fixation device which is adapted to the diameter of the medical instrument, thus providing a highly flexible and improved instrument fixation device.

The actuation device may be a self-locking device, which may be configured for locking the medical instruments, the sub-set of positions in the locked configuration being continuous or discontinuous positions, upon upon shifting the configuration of the instrument fixation device between the open and closed. By "continuous" is meant that the position of the first and/or second opposing clamping surfaces is determined by the diameter of the medical instrument and a frictional engagement between the medical instrument and the first and second opposing clamping surfaces. With "discontinuous" is meant that the positions of the first and second opposing clamping surfaces are predetermined positions.

The actuation member comprises a first and a second cooperating threaded member.

The actuation member comprises a male threaded member and a female threaded member being engaged with each other, and wherein the instrument fixation device is shifted between the open configuration and the closed configuration by rotation of the male threaded member relative to the female threaded member. An advantage of such actuation member may be that it is easy to handle for the surgical staff and that it requires a relatively constant and low actuation pressure in the transverse direction, which has been found reducing the risk of undesired displacement of the medical instrument when shifting between the open and the close configuration.

The first clamping surface of the instrument fixation device may constitute of an end portion of the male threaded member. The female threaded member may consequently be a threaded aperture extending, in the vertical direction, from an outer surface of the instrument fixation device and into the instrument receiving opening, the female threaded member being configured for threaded engagement with the male threaded member. Such configuration enables medical instrument of a wide variety of sizes to be fixed with the instrument fixation device and a constant and low actuation pressure to be used when shifting between the closed and the open configuration.

The instrument fixation device comprises a first clamping section and a second clamping section, the first and the second clamping sections being opposing sections forming together the instrument receiving opening, the first clamping section comprising the first opposing clamping surface and the second clamping section comprising the second opposing clamping surface of the instrument fixation device, wherein the male threaded member is connected to the first clamping section and the female threaded member is connected to the second clamping section. The female threaded member and the male threaded member may be arranged externally with respect to the instrument receiving opening and a main portion of the instrument fixation device.

The trocar housing connector of the instrument fixation device may enable a reversible connection with the trocar housing, such as a snap-fit connection, a threaded connection or a press-fit connection. This enhances the flexibility of the instrument fixation device, which may be connected and reconnected with trocars of different position during surgery in a simple manner.

According to a second aspect, the present disclosure relates to a trocar fixation assembly comprising a trocar fixation device for holding a trocar during use and an instrument fixation device for holding a medical instrument inserted into the trocar. The trocar extending in a longitudinal direction and a transverse direction and comprises a trocar cannula and a trocar housing. The trocar fixation device comprises a skin attachment element, comprising an opening configured for receiving the trocar during use and being disposed about a longitudinal center line, and a trocar support member configured for hold the trocar in position, as seen in the transverse direction of the trocar. The instrument fixation device comprises a trocar housing connector enabling connection between the instrument fixation device and the trocar housing. The instrument fixation device comprises a first side intended to face the trocar housing during use and a second side being an opposite side to the first side. The instrument fixation device furthermore comprises an instrument receiving opening extending in a longitudinal direction of the instrument fixation device between the first and the second side of the instrument fixation device. The instrument fixation device has an open position allowing the medical instrument to move freely in the longitudinal direction and a closed position wherein the medical instrument is restrained from movement in the longitudinal direction by clamping of the medical instrument between a first and second opposing clamping surface of the instrument fixation device, such that a distance between the first and the second opposing clamping surfaces is less when in the closed position and with a clamping force being directed in a transverse direction, being perpendicular to the longitudinal direction.

A trocar fixation assembly according to the second aspect greatly facilitates the minimally invasive surgical procedure for the surgeon and for the surgical staff.

The trocar fixation assembly comprises a trocar fixation device which may be attached to the skin of the patient by means of the skin attachment element and a trocar support member. The skin attachment element may comprise a first side being the skin facing side provided with for example a hydrophobic gel, such as silicon, and a second side, being the opposed side to the first side. The trocar support member may be arranged on, such as be connected to, the second side of the skin attachment element at the opening of skin attachment element. The trocar support member may alternatively be an integrated part of the skin attachment element. The trocar support member is configured for holding the trocar in position, as seen in the transverse direction of the trocar.

The trocar is inserted into the patient via the opening in the skin attachment element and is held in position during surgery. This allows for the surgeon to leave a first trocar in the trocar fixation assembly and proceed with a second trocar (inserted into a second trocar fixation assembly) utilizing a different medical instrument.

The first trocar may for example be utilized for inserting a medical instrument for holding, for example, tissue or part of an intestine by means of a retractor. The trocar fixation assembly comprises an instrument fixation device for holding a medical instrument inserted into the trocar. This means that undesired movement of the first trocar with the medical instrument may be prevented by the trocar fixation device and undesired movement of the medical instrument in the trocar, as seen in the longitudinal direction, may be prevented by the instrument fixation device, without the need of surgical staff needing to hold the trocar and the medical instrument in position, which may be challenging ergonomically. The trocar fixation assembly according to the present disclosure thus greatly reliefs the burden for the surgical staff in performing the minimally invasive surgery and

This is beneficial since it prevents undesired movement of a trocar inside a body cavity of the patient (which may be risky), but still allows the surgeon to adjust the position of the trocar in a situation specific manner.

The instrument fixation device comprises an actuation member configured for gradually moving, continuously or discontinuously, the first and second opposing clamping surface(s) in the transverse direction between the open configuration and the closed configuration.

The actuation member may be configured for gradually moving the first and/or second opposing clamping surface(s) by means of a translational movement of the actuation member being in the transverse direction of the instrument fixation device and wherein the instrument fixation device may hold elongated portions of medical instruments with varying diameters by varying the distance between the first and the second opposing clamping surfaces when the instrument fixation device is in the closed position.

The trocar support member of the trocar fixation device may be shifted between a first configuration, in which the trocar is held in a straight configuration and coincides with the longitudinal center line, and a second configured, wherein the trocar is held in a slanted configuration, in which a longitudinal axis of the trocar is arranged at an angle, α, with respect to the longitudinal center line.

This allows the surgeon to adjust the angular position of the trocar depending on his/her position in the room and depending on the specific surgical situation. Furthermore, the fact that the trocar is movable and may be held in a straight configuration and an angled configuration may allow the surgeon to change the position of the trocar in a simplified and more ergonomic manner. The surgeon does not need to lean over the patient or change position in the room in order to operate through the trocars, e.g. by inserting a variety of surgical tools into the trocar cannulas.

The trocar housing connector of the instrument fixation device may enable a reversible connection with the trocar housing, such as a snap-fit connection, a threaded connection or a press-fit connection.

The trocar fixation device may have an open configuration allowing the trocar to move freely in the longitudinal direction and a closed configuration wherein the trocar is restrained from movement in the longitudinal direction, by clamping of the trocar. The locking mechanism may for example be a screw mechanism or a lathe chuck mechanism.

The trocar fixation assembly may comprise a medical dressing, the medical dressing comprising an aperture configured to encircle the trocar during use.

The skin attachment element may comprise a base member surrounding the bottom surface of the trocar support member.

The base member is arranged to contact the skin of the patient, i.e. the skin circumventing the incision.

The medical dressing may comprise a centrally disposed aperture configured to encircle the trocar when inserted into the patient.

The medical dressing may encircle the trocar support member such that essentially no gaps are formed between the fixation device and the dressing. This is to avoid potential entry of contaminants.

The medical dressing may be arranged to overlap the base member of the skin-attachment element. Accordingly, the parts of the dressing extending beyond the contour of the base member of the skin attachment assembly may be adhesively attached to the skin. Furthermore, the provision of the base member secures that no gaps between the trocar support member and the dressing are formed.

The medical dressing may be arranged between the base member and the trocar support member.

Accordingly, the medical dressing may be clamped between the base member and the trocar support member during use. The medical dressing may be detachably or fixedly attached to the base member.

During use, the medical dressing is typically fixed between the base member and the trocar support member. After surgery, when the trocar is to be exerted from the body cavity and the trocar fixation assembly is to be removed, each of the components may be removed separately, or together, if desired.

The medical dressing extends uninterrupted around the trocar support member.

A tight seal is thereby provided, and the sterility is improved in the area circumventing the surgical site since the dressing contains no cuts or openable parts.

The medical dressing may comprise a backing layer and an adhesive skin contact layer.

The adhesive skin contact layer secures a tight fit to the skin, and the dressing is fixed in place in the area circumventing the surgical site (and the fixation device). Blood and body fluids exuded from the surgical site may be evaporated through the backing layer.

The adhesive skin contact layer may comprise a silicone based adhesive.

A silicone based adhesive is skin-friendly and gentle to the skin. The silicone based adhesive allows for the dressing to be removed in a gentle manner from the skin without causing trauma and without interfering with the incision.

The medical dressing may comprise an absorbent pad between the backing layer and the adhesive skin contact layer.

An absorbent pad is beneficial to absorb the blood arising from the surgical intervention. It is desirable to prevent blood from accumulating at the skin, and instead secure removal and proper handling of the blood and body fluids by means of the absorbent pad. Furthermore, contaminating microorganisms are prevented from accumulating at the skin, and the sterility is thereby improved.

The absorbent pad may comprise a polyurethane foam.

A polyurethane foam is beneficial since it is capable of absorbing large amounts of fluids, and also has a pressure relieving effect. The pressure relieving effect is beneficial to prevent pressure ulcers from occurring. A dressing comprising a polyurethane foam is also conformable.

According to a third aspect, the present disclosure relates to a trocar system comprising a trocar, the trocar comprising a trocar cannula and a trocar housing. The trocar system furthermore comprises a trocar fixation assembly according to the second aspect.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF DRAWINGS

The various aspects of the present disclosure, including its particular features and advantages, will be readily understood from the following detailed description and the accompanying drawings, in which:
Figure 1a-1b illustrate an instrument fixation device according to an exemplary embodiment of the present disclosure;
Figure 1c is an exploded view of a trocar fixation assembly according to an exemplary embodiment of the present disclosure associated with a trocar
Figure 2a-2d illustrate the trocar fixation assembly according to Fig. 1c; and
Figure 3a-3b illustrate an instrument fixation device according to an exemplary embodiment of the present invention.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like reference number denote similar features throughout the figures. Reference numbers may be omitted in some figures for better visibility, in which case reference is made to the other figures.

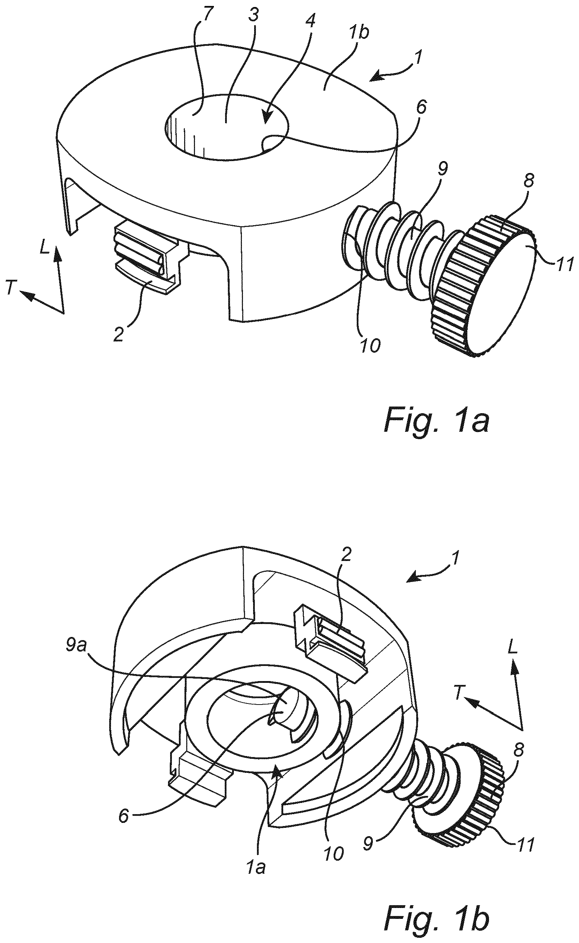

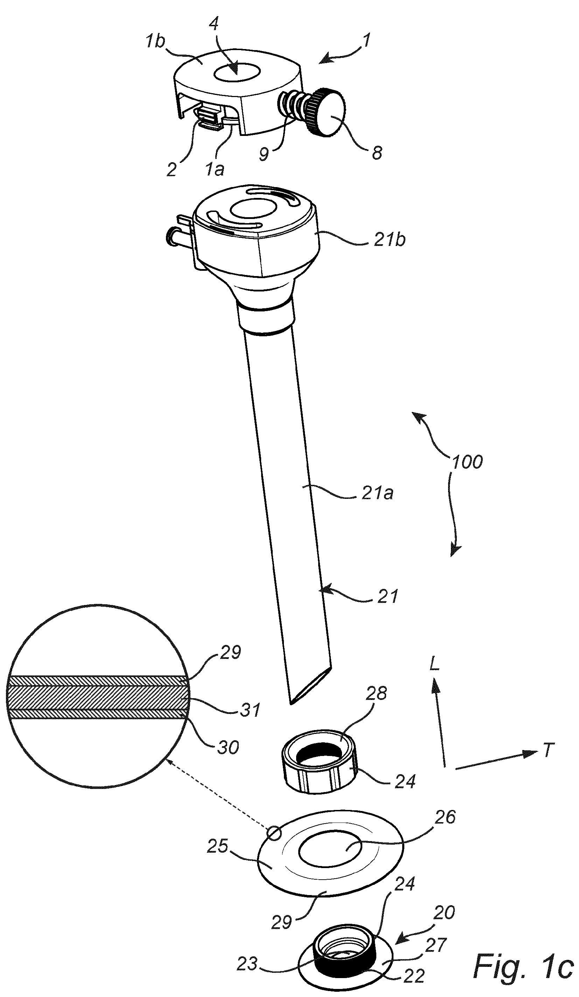

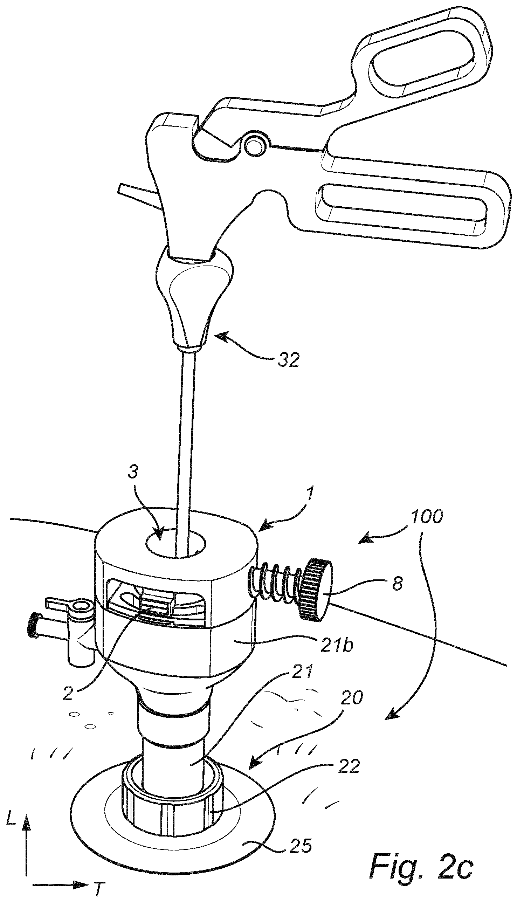

With reference to Figs 1a and 1b, an instrument fixation device 1 according to an exemplary embodiment of the present disclosure is conceptually illustrated. The instrument fixation device 1 is configured for holding a medical instrument 32 (shown in Fig. 2c-2e) in a trocar system. The trocar system comprises a trocar cannula 21a and a trocar housing 21b (shown in Fig. 1c). The instrument fixation device 1 comprises a trocar housing connector 2 adapted for connection between the instrument fixation device 1 and the trocar housing 21b. The trocar housing connector 2 of the instrument fixation device 1 enables a reversible connection with the trocar housing 21b. In Figs. 1a and 1b, the reversible connection is illustrated as a snap-fit connection. However, the connection may alternatively by a threaded connection or a press-fit connection.

The instrument fixation device 1 comprises a first side 1a intended to face the trocar housing 21b during use and a second side 1b being an opposite side to the first side 1a. The instrument fixation device 1 comprises an instrument receiving opening 3 extending in a longitudinal direction L of the instrument fixation device 1 between the first and the second side 1a, 1b of the instrument fixation device 1.

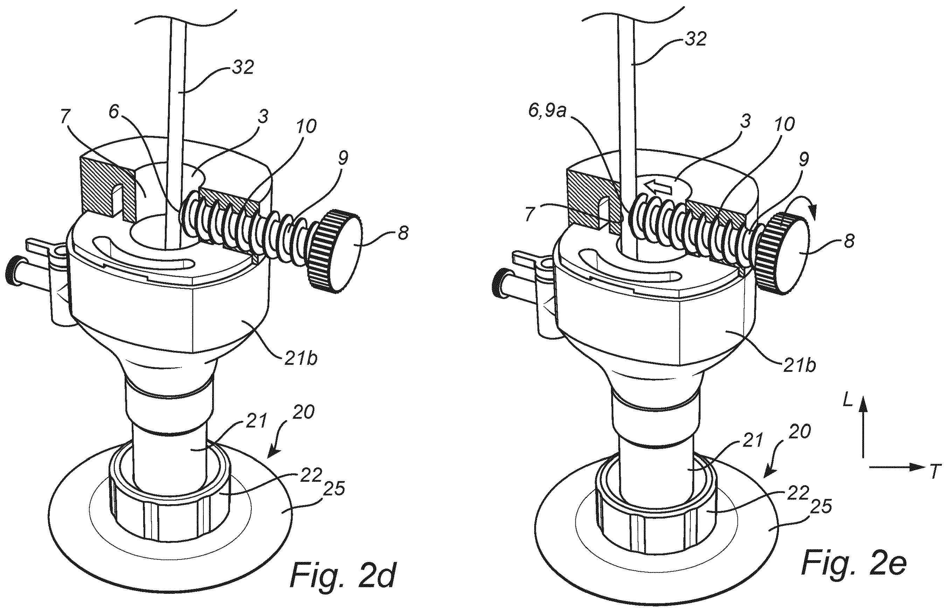

The instrument fixation device 1 has an open configuration 4, as illustrated in Figs. 1a and 1b, allowing the medical instrument to move freely in the longitudinal direction L (shown in Fig. 2d) and a closed configuration 5 (shown in Fig. 2e) wherein the medical instrument is restrained from movement in the longitudinal direction L by clamping of the medical instrument 32 between a first and a second opposing clamping surface 6,7 of the instrument fixation device 1 with a clamping force being directed in a transverse direction T. The transverse direction T is perpendicular to the longitudinal direction L. The instrument fixation device 1 comprises an actuation member 8 for shifting the configuration of the instrument fixation device 1 between the open configuration 4 and the closed configuration 5, by means of a translational movement of the actuation member 8, the translational movement being in the transverse direction T of the instrument fixation device 1. In the exemplary embodiment shown in Figs. 1a and 1b, the actuation member 8 comprises an actuation member knob 11, a first and a second cooperating threaded member in the form of a male threaded member 9 and a female threaded member 10 being threadedly engaged with each other. An end portion 9a of the male threaded member 9, arranged at an opposite end portion with respect to the actuation member knob 11, forms the first clamping surface 6 of the instrument fixation device 1. The instrument fixation device 1 may shifted between the open configuration 4 and the closed configuration 5 by rotating the actuation member knob 11, and thus the male threaded member 9 relative to the female threaded member 10.

The actuation member 8 is a mechanical linear actuator configured for gradually moving the first opposing clamping surface 6 in the transverse direction T by converting a rotary motion into linear motion such that the first clamping surface 6, here the end portion 9a of the male threaded member 9, approaches the second clamping surface 7 of the instrument fixation device 1. The female threaded member 10 is a threaded aperture extending, in the transverse direction T, from an outer surface of the instrument fixation device 1 and into the instrument receiving opening 3. Such configuration enables medical instrument of a wide variety of sizes to be fixed with the instrument fixation device and a constant and low actuation pressure to be used when shifting between the open and the closed configuration.

As used herein, the term "trocar" means a device through which a minimal invasive surgery can be accomplished. The trocar typically comprises at least a cannula; i.e. a hollow tube shaped member, a gas-tight valve and a trocar housing. The trocar allows for the entry of surgical instruments, such as graspers, scissors, staplers, cameras etc.

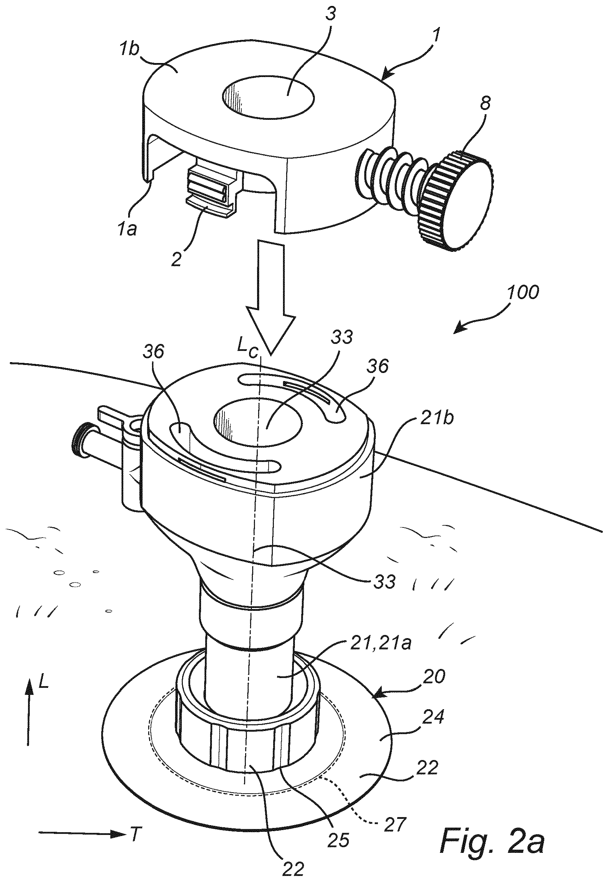

Fig. 1c is an exploded view of a trocar fixation assembly 100 according to the present disclosure. The trocar fixation assembly 100 comprises a trocar fixation device 20 for holding a trocar 21 during use and an instrument fixation device 1 for holding a medical instrument 32 (as illustrated in Figs. 2c-2e) inserted into the trocar 21. The trocar 21 extends in a longitudinal direction L and a transverse direction T. The trocar fixation device 20 comprises a skin attachment element 22, comprising an opening 23 configured for receiving the trocar 21 during use and being disposed about a longitudinal center line Lc (see Fig. 2a), and a trocar support member 24 configured for hold the trocar 21 in position, as seen in the transverse direction T of the trocar 21. The trocar fixation assembly 20 illustrated in Fig. 1c comprises a medical dressing 25 which is optional to the present invention. The medical dressing 25 comprises an aperture 26 configured to encircle the trocar 21 during use. The aperture 26 may be a centrally disposed aperture configured to encircle the trocar 21 when inserted into the patient.

As used herein, the term "trocar fixation assembly" means an assembly comprising at least a trocar fixation device and an instrument fixation device. The trocar fixation assembly is to be used in conjunction with a trocar. More particularly, the trocar fixation assembly is used to receive and engage with a trocar cannula.

The medical dressing may be fixedly or detachably attached to the trocar support member. The medical dressing is typically arranged to extend around the contour of the trocar support member and to be arranged in contact with the skin of the patient.

The term "skin attachment element" means an element adapted to be arranged in contact with the skin of a patient. Typically, the skin attachment element is arranged in an area of the skin surrounding an incision. Accordingly, after an incision has been made in the skin, the skin attachment element is typically arranged to surround the incision and facilitate the positioning and insertion of a trocar.

The skin attachment element 22 may comprise a base member 27 surrounding the bottom surface of the trocar support member 24. The base member 27 is arranged to contact the skin of the patient, i.e., the skin circumventing the incision.

The term "trocar support member" means a member adapted to be in contact with the trocar and to hold and support the trocar in a predefined position, such that unwanted movements of the trocar, as seen in the transverse direction, is prevented. The trocar support member may be a separate part from or an integrated part with the skin attachment element. When the trocar support member is a separate part with respect to the skin attachment element, these may be connectable with each other with cooperating connection elements.

The medical dressing may encircle the trocar support member such that essentially no gaps are formed between the fixation device and the dressing. This is to avoid potential entry of contaminants.

The medical dressing 25 may be arranged to overlap the base member 27 of the skin-attachment element 22. Accordingly, the parts of the dressing 25 extending beyond the contour of the base member 27 of the skin attachment element 22 may be adhesively attached to the skin. Furthermore, the provision of the base member 27 secures that no gaps between the trocar fixation device 20 and the dressing 25 are formed.

Optionally, and as disclosed in Fig. 1c, the trocar support member 24 comprises an upper support portion 28. In this figure, the upper support portion 28 and the trocar support member 24 are connected by means of a screw mechanisms. The trocar fixation device 20 may have an open configuration allowing the trocar 21 to move freely in the longitudinal direction L and a closed configuration wherein the trocar 21 is restrained from movement in the longitudinal direction L, by clamping of the trocar 21. The medical dressing 25 may be arranged between the base member 27 and the upper support portion 28 of the trocar support member 24.

Accordingly, the medical dressing 25 may be clamped between the base member 27 and the upper support portion 28 during use. The medical dressing may be detachably or fixedly attached to the base member.

The medical dressing 25 may extend uninterrupted around the trocar support member 24 as disclosed in Fig. 1c. A tight seal is thereby provided, and the sterility is improved in the area circumventing the surgical site since the dressing contains no cuts or openable parts. The medical dressing 25 may comprise a backing layer 29 and an adhesive skin contact layer 30, as may be seen in the zoomed-in view of the medical dressing 25.

The adhesive skin contact layer 30 secures a tight fit to the skin, and the dressing 25 is fixed in place in the area circumventing the surgical site (and the fixation device). Blood and body fluids exuded from the surgical site may be evaporated through the backing layer 29. The adhesive skin contact layer 30 may comprise a silicone-based adhesive. A silicone-based adhesive is skin-friendly and gentle to the skin. The silicone-based adhesive allows for the dressing 25 to be removed in a gentle manner from the skin without causing trauma and without interfering with the incision. As illustrated in the zoomed-in view of the medical dressing 25, the medical dressing 25 may furthermore comprise an absorbent pad 31 between the backing layer 29 and the adhesive skin contact layer 30.

An absorbent pad 31 is beneficial to absorb the blood arising from the surgical intervention. It is desirable to prevent blood from accumulating at the skin, and instead secure removal and proper handling of the blood and body fluids by means of the absorbent pad 31. Furthermore, contaminating microorganisms are prevented from accumulating at the skin, and the sterility is thereby improved. The absorbent pad 31 may comprise a polyurethane foam.

A polyurethane foam is beneficial since it is capable of absorbing large amounts of fluids, and also has a pressure relieving effect. The pressure relieving effect is beneficial to prevent pressure ulcers from occurring. A dressing comprising a polyurethane foam is also conformable.

Fig. 2a illustrates the trocar fixation assembly 100 of Fig. 1c when the trocar fixation device 20 is attached to the skin of a patient and the trocar 20 is inserted into the patent and supported by the trocar fixation device 20. The trocar support member 24 of the trocar fixation device 20 holds the trocar in a straight configuration, coinciding with the longitudinal center line Lc.

The instrument fixation device 1 comprises a trocar housing connector 2 adapted for connection between the instrument fixation device 1 and the trocar housing 21b. The trocar housing connector 2 of the instrument fixation device 1 is a snap-fit connector enabling a reversible connection with the trocar housing 21b. The trocar housing 21b comprises a corresponding fixation device connector 36, adapted to engage with the trocar housing connector 2 of the instrument fixation device 1, here in the form of a respective slit in the trocar housing connector 21b.

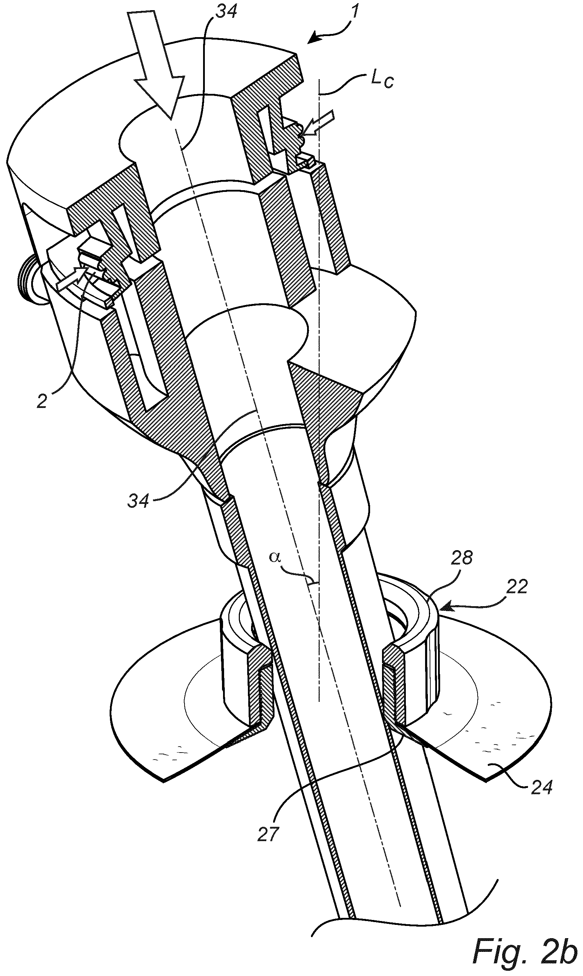

As illustrated in Figs. 2a and 2b, the trocar support member 24 of the trocar fixation device 20 may be shifted between a first configuration 33, in which the trocar 21 is held in a straight configuration and coincides with the longitudinal center line Lc (shown in Fig. 2a), and a second configured, wherein the trocar 21 is held in a slanted configuration 34, in which a longitudinal axis of the trocar 21 is arranged at an angle, α, with respect to the longitudinal center line Lc, as illustrated in Fig. 2b.

This allows the surgeon to adjust the angular position of the trocar depending on his/her position in the room and depending on the specific surgical situation. Furthermore, the fact that the trocar is movable and may be held in a straight configuration and an angled configuration may allow the surgeon to change the position of the trocar in a simplified and more ergonomic manner. The surgeon does not need to lean over the patient or change position in the room in order to operate through the trocars, e.g. by inserting a variety of surgical tools into the trocar cannulas.

Figs. 2c-2e illustrate the trocar fixation assembly 100 according to Fig. 1c, comprising the instrument fixation device 1 and the trocar fixation device 20. The instrument fixation device 1 is connected to the trocar housing 21b by means of the trocar housing connector 2. The instrument fixation device 1 comprises an instrument receiving opening 3 for receiving a medical instrument 32 being inserted into the trocar 21. In Figs. 2c and 2d, the instrument fixation device is in an open configuration allowing the medical instrument to move freely in the longitudinal direction L. In Fig. 2e, the instrument fixation device 1 is in a closed configuration wherein the medical instrument 32 is restrained from movement in the longitudinal direction L by clamping of the medical instrument 32 between the first and second opposing clamping surface 6,7 of the instrument fixation device 1 with a clamping force being directed in a transverse direction T, the transverse direction T being perpendicular to the longitudinal direction L. The instrument fixation device 1 may be shifted between the open configuration and the closed configuration by rotating the actuation member 8 such that the first clamping surface 6, here corresponding to the end portion 9a of the male threaded member 9, clamps, and thus frictionally engages, the medical instrument 32 between the first clamping surface 6 and the second clamping surface 7, the second clamping surface 7 here corresponding to an inner wall of the instrument receiving opening 3.

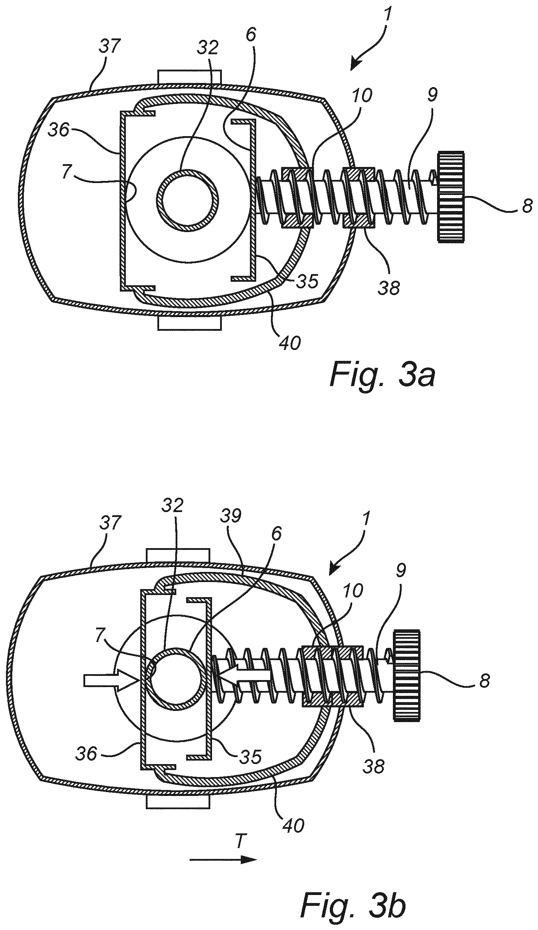

Figs. 3a and 3b illustrate an alternative instrument fixation device 1 according to the present invention. The instrument fixation device 1 comprises in this embodiment, a first clamping section 35, a second clamping section 36 and an instrument fixation device housing 37, the first and the second clamping sections 35,36 being opposing sections forming together the opening 3 of the instrument fixation device 1 when being brought together. The instrument fixation device housing 37 comprises a trocar housing connector (not shown). As may be seen in the figures, a width of the second clamping section 36 is greater than a width of the first section 35, thus allowing the instrument fixation device 1 to hold smaller-sized medical instruments in the closed configuration. The first clamping section 35 comprises a first opposing clamping surface 6 on an inner surface thereof and a male threaded member 9 is connected to an outer surface of the first clamping section 35. The first clamping section 35 and the second clamping section 36 are inner sections and housed in the instrument fixation device housing 37. The instrument fixation device housing 37 is connected to the male threaded member 9 vi a female threaded housing member 38. The second clamping section 36 comprises a second opposing clamping surface 7 on an inner surface thereof and a female threaded member 10 is connected to the second clamping section 36 via a first and a second support arm 39,40. The male threaded member 9 and the female threaded member 10 are threadedly engaged with each other and upon rotation of an actuation member 8, the male threaded member 9 will gradually move in the transverse direction T and pull the first and second clamping sections 35,36 such that the first and second opposing clamping surfaces 6,7 approaches each other, as illustrated in Fig. 3a, until the medical instrument 32 is clamped between the first and the second clamping surfaces 6,7 and frictionally engaged in the closed position, as illustrated in Fig. 3b.
Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. An instrument fixation device (1) for holding a medical instrument (32) in a trocar system (21), the trocar system (21) comprising a trocar cannula (21a) and a trocar housing (21b), the instrument fixation device (1) comprising a trocar housing connector (2) adapted for connection between the instrument fixation device (1) and the trocar housing (21b), the instrument fixation device (1) comprising a first side (1a) intended to face the trocar housing (21b) during use and a second side (1b) being an opposite side to the first side (1a), the instrument fixation device (1) comprising an instrument receiving opening (3) extending in a longitudinal direction (L) of the instrument fixation device (1) between the first and the second side (1a,1b) of the instrument fixation device (1), the instrument fixation device (1) having an open configuration allowing the medical instrument (32) to move freely in the longitudinal direction (L) and a closed configuration wherein the medical instrument (32) is restrained from movement in the longitudinal direction (L) by clamping of the medical instrument (32) between a first and second opposing clamping surface (6,7) of the instrument fixation device (1) with a clamping force being directed in a transverse direction (T) being perpendicular to the longitudinal direction (L), the instrument fixation device (1) comprising an actuation member (8) configured for shifting the configuration of the instrument fixation device (1) between the open configuration and the closed configuration, by means of a translational movement of the actuation member (8), the translational movement being in the transverse direction (T) of the instrument fixation device (1) and wherein the closed configuration of the instrument fixation device (1) includes a sub-set of positions with varying distances between the first and the second opposing clamping surface (6,7) in each of the positions in the sub-set of positions,
**characterized in that** the actuation member (8) comprises a male threaded member (9) and a female threaded member (10) being engaged with each other, and wherein the instrument fixation device (1) is shifted between the open configuration and the closed configuration by rotating the male threaded member (9) relative to the female threaded member (10) and wherein the instrument fixation device (1) comprises a first clamping section (35) and a second clamping section (36), the first and the second clamping sections (35,36) being opposing sections forming together the opening (3), the first clamping section (35) comprising the first opposing clamping surface (6) and the second clamping section (36) comprising the second opposing clamping surface (7) of the instrument fixation device (1), wherein the male threaded member (9) is connected to the first clamping section (35) and the female threaded member (10) is connected to the second clamping section (36), wherein the actuation device (8) is configured for gradually moving, continuously or discontinuously, the first and second opposing clamping surface(s) (6,7) in the transverse direction (T).

2. The instrument fixation device (1) according to claim 1, wherein the first clamping surface (6) of the instrument fixation device (1) constitutes of an end portion (9a) of the male threaded member (9).

3. The instrument fixation device (1) according to any one of the preceding claims, wherein the trocar housing connector (21b) of the instrument fixation device (1) enables a reversible connection with the trocar housing (21b), such as a snap-fit connection, a threaded connection, or a press-fit connection.

## Patentansprüche

1. Instrumentenbefestigungsvorrichtung (1) zum Halten eines medizinischen Instruments (32) in einem Trokarsystem (21), wobei das Trokarsystem (21) eine Trokarkanüle (21a) und ein Trokargehäuse (21b) umfasst, wobei die Instrumentenbefestigungsvorrichtung (1) einen Trokargehäuseverbinder (2) umfasst, der für eine Verbindung zwischen der Instrumentenbefestigungsvorrichtung (1) und dem Trokargehäuse (21b) ausgelegt ist, wobei die Instrumentenbefestigungsvorrichtung (1) eine erste Seite (1a), die während des Gebrauchs dem Trokargehäuse (21b) zugewandt sein soll, und eine zweite Seite (1b) umfasst, bei der es sich um eine der ersten Seite (1a) gegenüberliegende Seite handelt, wobei die Instrumentenbefestigungsvorrichtung (1) eine Instrumentenaufnahmeöffnung (3) umfasst, sie sich in einer Längsrichtung (L) der Instrumentenbefestigungsvorrichtung (1) zwischen der ersten und der zweiten Seite (1a ,1b) der Instrumentenbefestigungsvorrichtung (1) erstreckt, wobei die Instrumentenbefestigungsvorrichtung (1) eine offene Gestaltung, in der sich das medizinische Instrument (32) frei in der Längsrichtung (L) bewegen lässt, und eine geschlossene Gestaltung aufweist, in der eine Bewegung des medizinischen Instruments (32) in der Längsrichtung (L) verhindert wird, indem das medizinische Instrument (32) zwischen einer ersten und zweiten gegenüberliegenden Klemmfläche (6, 7) der Instrumentenbefestigungsvorrichtung (1) mit einer Klemmkraft eingespannt wird, die in eine Querrichtung (T) gerichtet ist, die senkrecht zur Längsrichtung (L) verläuft, wobei die Instrumentenbefestigungsvorrichtung (1) ein Betätigungselement (8) umfasst, das zum Wechseln der Gestaltung der Instrumentenbefestigungsvorrichtung (1) zwischen der offenen Gestaltung und der geschlossenen Gestaltung mittels einer Translationsbewegung des Betätigungselements (8) ausgelegt ist, wobei die Translationsbewegung in der Querrichtung (T) der Instrumentenbefestigungsvorrichtung (1) erfolgt und wobei die geschlossene Gestaltung der Instrumentenbefestigungsvorrichtung (1) eine Teilmenge von Positionen mit unterschiedlichem Abstand zwischen der ersten und der zweiten gegenüberliegenden Klemmfläche (6, 7) in jeder der Positionen in der Teilmenge von Positionen aufweist,
**dadurch gekennzeichnet, dass** das Betätigungselement (8) ein Außengewindeelement (9) und ein Innengewindeelement (10) umfasst, die ineinandergreifen, und wobei zwischen der offenen Gestaltung und der geschlossenen Gestaltung der Instrumentenbefestigungsvorrichtung (1) durch Drehen des Außengewindeelements (9) bezogen auf das Innengewindeelement (10) gewechselt wird und wobei die Instrumentenbefestigungsvorrichtung (1) einen ersten Klemmabschnitt (35) und einen zweiten Klemmabschnitt (36) umfasst, wobei der erste und der zweite Klemmabschnitt (35, 36) gegenüberliegende Abschnitte sind, die gemeinsam die Öffnung (3) bilden, wobei der erste Klemmabschnitt (35) die erste gegenüberliegende Klemmfläche (6) umfasst und der zweite Klemmabschnitt (36) die zweite gegenüberliegende Klemmfläche (7) der Instrumentenbefestigungsvorrichtung (1) umfasst, wobei das Außengewindeelement (9) mit dem ersten Klemmabschnitt (35) verbunden ist und das Innengewindeelement (10) mit dem zweiten Klemmabschnitt (36) verbunden ist, wobei die Betätigungselement (8) so ausgelegt ist, dass sie die erste und zweite gegenüberliegende Klemmfläche (6, 7) kontinuierlich oder diskontinuierlich nach und nach in der Querrichtung (T) bewegt.

2. Instrumentenbefestigungsvorrichtung (1) nach Anspruch 1, wobei die erste Klemmfläche (6) der Instrumentenbefestigungsvorrichtung (1) aus einem Endabschnitt (9a) des Außengewindeelements (9) besteht.

3. Instrumentenbefestigungsvorrichtung (1) nach einem der vorhergehenden Ansprüche, wobei der Trokargehäuseverbinder (21b) der Instrumentenbefestigungsvorrichtung (1) eine reversible Verbindung mit dem Trokargehäuse (21b) ermöglicht, beispielsweise eine Rastverbindung, eine Schraubverbindung oder eine Einpressverbindung.

## Revendications

1. Dispositif de fixation d'instrument (1) pour maintenir un instrument médical (32) dans un système de trocart (21), le système de trocart (21) comprenant une canule de trocart (21a) et un boîtier de trocart (21b), le dispositif de fixation d'instrument (1) comprenant un connecteur de boîtier de trocart (2) adapté pour la connexion entre le dispositif de fixation d'instrument (1) et le boîtier de trocart (21b), le dispositif de fixation d'instrument (1) comprenant un premier côté (la) destiné à faire face au boîtier de trocart (21b) pendant l'utilisation et un second côté (1b) opposé au premier côté (1a), le dispositif de fixation d'instrument (1) comprenant une ouverture de réception d'instrument (3) s'étendant dans une direction longitudinale (L) du dispositif de fixation d'instrument (1) entre le premier et le second côté (1a, 1b) du dispositif de fixation d'instrument (1), le dispositif de fixation d'instrument (1) ayant une configuration ouverte permettant à l'instrument médical (32) de se déplacer librement dans le sens longitudinal (1) et une configuration fermée, l'instrument médical (32) étant empêché de se déplacer dans la direction longitudinale (L) par serrage de l'instrument médical (32) entre une première et une seconde surfaces de serrage opposées (6, 7) du dispositif de fixation d'instrument (1), une force de serrage étant dirigée dans une direction transversale (T) perpendiculaire à la direction longitudinale (L), le dispositif de fixation d'instrument (1) comprenant un élément d'actionnement (8) configuré pour permuter la configuration du dispositif de fixation d'instrument (1) entre la configuration ouverte et la configuration fermée, au moyen d'un mouvement de translation de l'élément d'actionnement (8), le mouvement de translation étant dans la direction transversale (T) du dispositif de fixation d'instrument (1) et la configuration fermée du dispositif de fixation d'instrument (1) comprenant un sous-ensemble de positions avec des distances variables entre la première et la seconde surface de serrage opposée (6, 7) dans chacune des positions du sous-ensemble de positions,
**caractérisé en ce que** l'élément d'actionnement (8) comprend un élément fileté mâle (9) et un élément fileté femelle (10) engagés l'un dans l'autre, et le dispositif de fixation d'instrument (1) étant permuté entre la configuration ouverte et la configuration fermée, par rotation de l'élément fileté mâle (9) par rapport à l'élément fileté femelle (10) et le dispositif de fixation d'instrument (1) comprenant une première section de serrage (35) et une seconde section de serrage (36), les première et seconde sections de serrage (35, 36) étant des sections opposées formant ensemble l'ouverture (3), la première section de serrage (35) comprenant la première surface de serrage opposée (6) et la seconde section de serrage (36) comprenant la seconde surface de serrage opposée (7) du dispositif de fixation d'instrument (1), l'élément fileté mâle (9) étant relié à la première section de serrage (35) et l'élément fileté femelle (10) étant relié à la seconde section de serrage (36), le dispositif d'actionnement (8) étant configuré pour déplacer progressivement, de manière continue ou discontinue, la première et la seconde surface(s) de serrage (6, 7) dans la direction transversale (T).

2. Dispositif de fixation d'instrument (1) selon la revendication 1, dans lequel la première surface de serrage (6) du dispositif de fixation d'instrument (1) consiste en une partie d'extrémité (9a) de l'élément fileté mâle (9).

3. Dispositif de fixation d'instrument (1) selon l'une quelconque des revendications précédentes, dans lequel le connecteur de boîtier de trocart (21b) du dispositif de fixation d'instrument (1) permet une connexion réversible avec le boîtier de trocart (21b), telle qu'une connexion à encliquetage, une connexion filetée ou une connexion à ajustement serré.
